# EUROPEAN PATENT APPLICATION

(11) **EP 4 725 962 A1**
(43) Date of publication of application: **15.04.2026**
(21) Application number: 24206264.4
(22) Date of filing: 11.10.2024
(51) Int. Cl.: C07K 14/78, C12N 5/00, C12N 5/074

(54) **PROCESS AND DEVICE FOR CULTIVATION OF IPSC**

(71) Applicant: Gottfried Wilhelm Leibniz Universität Hannover, 30167 Hannover (DE); Helmholtz Zentrum München, 85764 Neuherberg (DE)
(72) Inventor: Pirmahboub, Hamidreza, Hannover (DE); Lee-Thedieck, Cornelia, Wunstorf (DE); Klein, Gerd, Tübingen (DE); Plettenburg, Prof. Dr., Oliver, Burgdorf (DE); Schertl, Dr., Peter, Gehrden (DE)
(74) Representative: Taruttis, Stefan Georg

(57) **Abstract**

The invention provides a process for cultivating iPSC adherent to a carrier having on its surface a covalently bound laminin peptide, which consist a specified amino acid sequence section at least 13 amino acids, of at least 14 amino acids or of 15 amino acids.

## Description

The present invention relates to a process for cultivating mammalian cells, e.g. induced pluripotent stem cells (iPSC), preferably human cells, the cells growing adherently on a carrier with covalently bound laminin peptides, to the use of the carrier with the laminin peptides for the cultivation process, to a process for production of the carrier with laminin peptides, and to the carrier with the laminin peptides obtainable by the process for production.

The process has the advantage of cultivating mammalian cells, preferably iPSC, in an adherently growing manner, preferably for at least 24 h, preferably for at least 48 h, more preferably for at least 72 h, e.g. for 1 to 5 days, or for 2 to 4 days. Further, the process has the advantage of exclusively cultivating adherent iPSC, i.e. cultivating iPSC in the absence of feeder cells. Further, the process can be performed in medium which is devoid of ingredients of animal origin, e.g. in serum-free medium, especially in medium consisting of synthetic ingredients.

### State of the art

US 2019/0211305 A1 describes cultivating pluripotent stem cells by using laminin 421, laminin 121 or fragments of the two laminins. The patent only mentions E8 fragments, which consist of a C-terminal fragment of an α chain from which the globular domains 4 and 5 have been removed, a C-terminal fragment of a β-chain and a C-terminal fragment of a γ-chain, resulting in a trimer.

For cultivating stem cells US 9 868 828 B2 describes the production of a three-dimensional matrix by electrospinning of a polymer and a substrate protein, e.g. mussel adhesive protein, presenting a bound peptide motif derived from extracellular matrix proteins or growth factors. As a peptide motif bound to the substrate protein of the electrospun polymer, laminin derived peptides are described.

### Object of the invention

It is an object of the invention to provide a process for cultivating iPSC such that they grow adherently to a carrier, wherein the carrier is preferably provided with peptides which are small enough to allow for efficient production by chemical synthesis. Generally, the cultivation shall use serum-free medium and be free from feeder cells.

### Description of the invention

In preliminary experiments it was found that iPSC can only be cultivated for an extended period adherently in a culture vessel, when the culture vessel was coated with compounds supporting adherent growth, e.g. in microtiter plates coated with exemplary protein mixtures, e.g. coated with basement membrane-like matrices like Matrigel or mixtures of different laminin proteins. iPSCs could not be cultured for extended periods, when the culture vessels were non-covalently coated by physisorption with short protein fragments, i.e. peptides. Therein, the coated culture vessel was prepared by adding an aqueous solution of the peptide to a microtiter plate of polystyrene, followed by incubation for some time, and then removing the solution containing unbound peptide to generate a coated vessel. The generation of a coated vessel, e.g. a well of a microtiter plate, by simply introducing a peptide in aqueous solution, incubating for some time and then removing the remaining solution of unbound peptide is regarded as a standard and usual practice for coating a vessel, e.g. a well of a microtiter plate, with a peptide. Generally herein, a peptide refers to any number of peptide molecules having the same amino acid sequence.

The invention achieves the object by the features of the claims and especially by providing a process for cultivating iPSC adherent to a carrier, the process comprising the steps of providing a carrier having on its surface a covalently bound laminin peptides, which consist of one of the following laminin peptides or combinations of at least two of those, the peptides having a sequence equality of at least 80%, preferably an equality of an amino acid sequence section at least 13 amino acids, of at least 14 amino acids or of 15 amino acids, the peptides consisting of SEQ ID NO: 1 to SEQ ID NO: 7 (SEQ ID NO: 1 WLSERPKLAPDAEDS, SEQ ID NO: 2 HTQSTSVDTNNPIYV, SEQ ID NO: 3 YMGSRQATGDYMGVS, SEQ ID NO: 4 KASRRSRQPARHPAC, SEQ ID NO: 5 RQHQGAEHPQPHTLF, SEQ ID NO: 6 SKAIQVFLLGGSRKR or a shorter version of SEQ ID NO: 6, namely SEQ ID NO: 7 ASKAIQVFLLGG) or a combination of at least two of these, preferably at least one of SEQ ID NO: 2, SEQ ID NO: 3, SEQ ID NO: 4, SEQ ID NO: 6, SEQ ID NO: 7, or a combination of at least two of these, wherein the peptide is directly covalently bound to the carrier or the peptide has an C-terminal spacer group which is covalently linked to the surface of the carrier followed by contacting a serum-free cultivation medium and iPSC with the carrier and incubating the carrier in contact with the cultivation medium and iPSC under cell culture conditions for at least 24 h, preferably for at least 48 h, more preferably for at least 72 h.

It was found that these peptides, and any combination of at least two of these, when covalently bound to the carrier, support iPSC in cell culture medium under cell culture conditions and result in adherent growth, e.g. for at least 48 h or at least 72 h, e.g. for 24 h to 72 h or longer, under static incubation conditions. The covalent linkage of at least one of the peptides to the carrier is due to the covalent bond of the peptide with the carrier, optionally with a spacer coupling the peptide with the carrier, e.g. a spacer which comprises a thioether group covalently bound to the carrier, e.g. a thioether group originating from a thioether group which is part of a coupling group that is covalently bound to the carrier.

Incubation can be under agitated conditions, e.g. with agitation of the culture medium by moving the medium relative to the carrier, preferably gently. Preferably, incubation is under static conditions, i.e. without mechanically agitating the medium contacting the carrier.

The cultivation process is devoid of feeder cells, i.e. the process is for cultivating iPSC exclusively, e.g. only iPSC in absence of other cells.

Preferably, the cultivation process uses serum-free culture medium, e.g. a culture medium devoid of animal or human blood serum, especially a culture medium containing synthetic and/or isolated ingredients only. It was found that the covalent binding of at least one of the peptides to a carrier, preferably of glass or plastics, allows adherent growth of iPSC for at least 48 h or at least 72 h also in serum-free medium, especially culture medium consisting of synthetic ingredients only.

Generally, cell culture conditions are known, e.g. 37°C in a 5% CO₂ atmosphere.

The carrier, of glass or plastics, may be a culture vessel, such that the adherently growing iPSC are arranged on the inner surface of the vessel, the inner surface has at least one covalently bound peptide and is covered by the culture medium. In a simple embodiment, the carrier is the bottom and/or the wall of a vessel, which is e.g. a culture plate or a well of a microtiter plate.

The carrier may be in the form of plates, tubes, rods, fibres, e.g. electrospun fibres or drawn fibers, beads or hollow spheres, which are arranged, e.g. fixed or movably arranged or suspended in the culture medium, in a culture vessel, which may be of glass, metal, or of plastics.

The plastics may be polycarbonate, polycaprolactone, polystyrene, polyethylene or polypropylene.

As an alternative to a solid of glass or plastics, the carrier may be a hydrogel, e.g, consist of or be derived from natural polymers, synthetic polymers or hybrid materials that combine synthetic and natural polymers. The natural biopolymers may be derived from polysaccharides including e.g. agarose, alginate, amylose, carrageenan, chitosan, carboxymethyl cellulose, chondroitin sulfate, cellulose, dextran, heparin, hyaluronan and pectin or from proteins like collagen, fibrin, gelatin, gelatin methacrylate, serum albumins and combinations thereof, preferably combinations of two. For biohybrid hydrogels, these polymers may be crosslinked with polyethylene glycol linkers of varying chain length or other synthetic linkers, preferably heparin crosslinked with starPEG. Alternatively, the hydrogels may also be derived from synthetic polymers such as polyethylene glycol), polyacrylamide, poly(vinyl alcohol) (PVA), poly(2-hydroxylethyl methacrylate) (PHEMA), zwitterionic polymers like polycarboxybetaines or combinations thereof preferably combinations of two.

The invention is now described in greater detail by examples and with reference to the figures, which show in
- Fig. 1a results for adhesion of iPSC to a solid surface carrier (peptide array) provided with attached peptides,
- Fig. 2 microscopic images of iPSC expressing OCT4 and SSEA4 after cultivation for 24 h on a solid surface carrier provided with peptides of the invention,
- Fig. 3 graph for relative expression of OCT4 by cells growing adherently on a solid surface carrier with bound peptides of the invention, and
- Fig. 4 a microscopic image of adherent iPSC on a hydrogel carrier with bound peptides of the invention.

### Example 1: Identification of peptides for adherent growth of iPSC

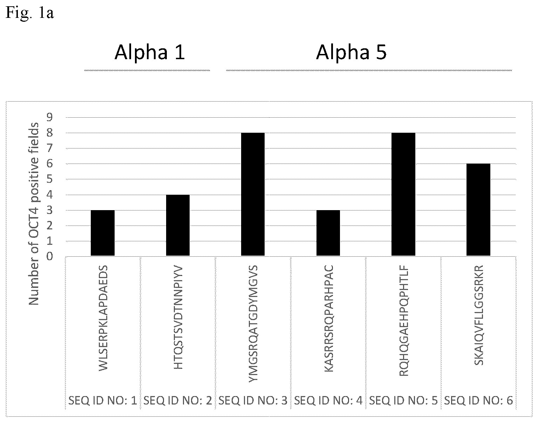

The peptides of SEQ ID NO: 1 to SEQ ID NO: 6 (Fig. 1a) were identified in an peptide assay, detecting binding of iPSC to individual sections of a solid surface carrier in the form of a glass plate. Therein, the peptides form a microarray of separate surface sections, each provided with peptides of one amino acid sequence. The peptides were chemically synthesized directly on the glass plate. Binding of iPSC to an individual section of the glass plate carrier was analysed optically, pluripotency as important stem cell property was determined via immune fluorescence detecting the pluripotency marker OCT4. In detail, the cells were allowed to adhere for 24 h to the glass-bound peptides and then nuclei were stained with DAPI (blue) and cells were immunostained for the pluripotency marker OCT4. Next, positively stained areas were optically detected and analyzed using the Mapix software.

The peptides consisted of sections of 15 amino acids of the G-domain of α-1 laminin and of α-5 laminin, with an overlap of 5 amino acids. In total, 197 peptides, including the peptides of SEQ ID NO: 1 to SEQ ID NO: 38 were synthesized and bound to the glass plate.

Based on the results of the peptide microarray, six peptides were chosen for iPSC cultivation in further experiments. Instead of the peptide SKAIQVFLLGGSRKR identified as adhesive in the screens, a shorter peptide SEQ ID NO: 7 A5G4 (ASKAIQVFLLGG) was used for further analyses, which had been identified in previous studies to be an adhesively active peptide.

Fig.1a shows the results of experiments to identify adhesive peptide sequences within laminin α-1 and α-5 chains via a peptide array. Adhesive peptides for iPS cells from the G-domain of the α-1 and α -5 chains of laminin are shown. 15 amino acid long peptides (98 in total each) of the α-1 and the α-5 G-domain were printed on slides and iPS cells were then allowed to adhere to the peptides for 24 h. OCT4 positive cells were visualized by immunofluorescence and positive fields were counted after 24 h of adhesion. The x-axis shows the peptide sequence and the y-axis the number of positive OCT4 signals of the respective peptide.

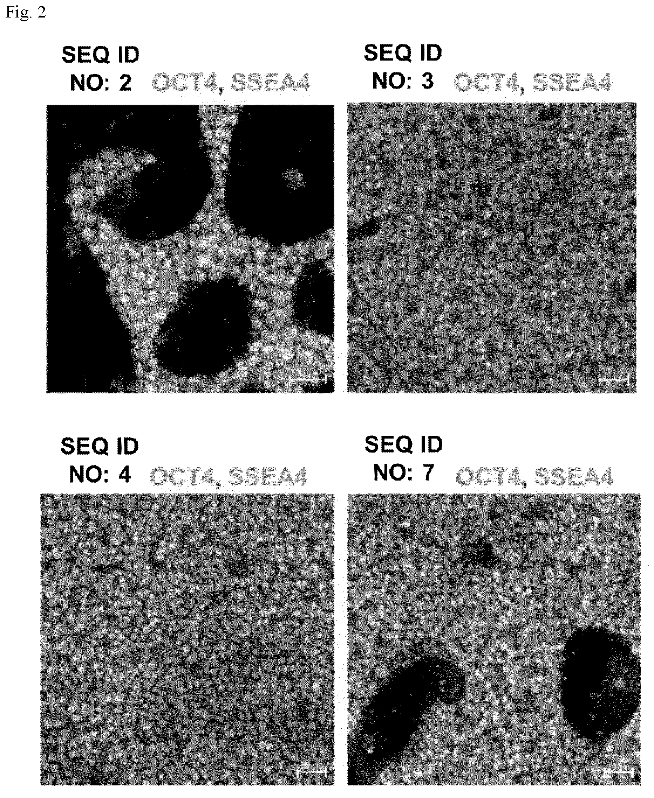

As a negative control, laminin peptides were physically adsorbed without covalent linkage in wells of a plastic microtiter plate by adding an aqueous solution of individual peptides and after incubation for at least 1 h at 37 °C, removing the solution and washing the wells with culture medium, resulting in non-covalent binding to the carrier (Fig. 2). Non-covalent binding of the peptide resulted in delamination of initially attached cell layers of the initially adhered cells over time, e.g. within 24 h in culture. Cell attachment was only stable for 24 hours, thereafter detachment occurred showing that immobilization of the peptides by adsorption without covalent linkage was only suitable for short term culture.

Fig. 2 shows representative images of immunofluorescence-stained iPSC that were cultured for 24 h on peptide-coated cell culture plastic. OCT4 and SSEA4 expression of iPSC was analysed by immunofluorescence staining after 24 h of culture on adhesive peptides of the G-domain of the laminin α-1 and α-5 chains which were coated on tissue culture plastic without covalent binding. The pluripotency markers OCT4 (red) and SSEA4 (green) can be seen. The cell nuclei were stained with DAPI (blue). In adhesive tests the four peptides SEQ ID NO: 2, SEQ ID NO: 3, SEQ ID NO: 4, SEQ ID NO: 6, and SEQ ID NO: 7 were shown to be adhesive peptides for iPSC . While cells adhered for short periods of time to such substrates prepared by physisorption of the peptides, they delaminated with time from the surface and thus, no longer-term culture was possible on these substrates. Scale bar: 50 µm. Peptides having other sequences were significantly less effective in this assay.

### Example 2: Cultivation of iPSC on solid carrier

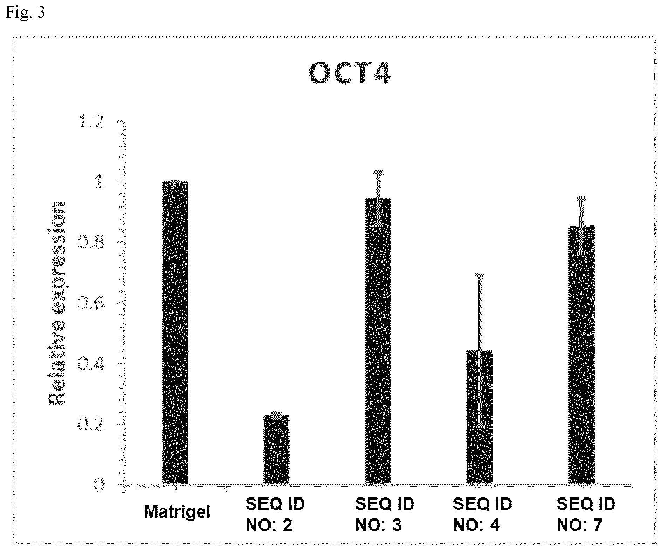

A carrier with covalently bound peptides was used, the peptides that proved to be adhesive (see Example 1) were covalently bound to a glass carrier. The peptides had the amino acid sequence of SEQ ID NO: 2, SEQ ID NO: 3, SEQ ID NO: 4 or SEQ ID NO: 7. The covalent linkage was generated by using peptides functionalized at their C-Terminus with a maleimide group and thiol-modified glass. In detail, glass slides were washed with ethanol for 10 min followed by rinsing in acetone and sonication in ultrapure water for 10 min, then drying under nitrogen flow. The glass slides were then placed inside an O₂ plasma generator (TePla 100-E from Technics Plasma GmbH, Germany) for 20 min, power at 200 W. After O₂ plasma treatment, a 0.46 mM solution of 3-mercapto propylsilatrane (MPS) in a 1:1 ethanol:H₂O mixture (1 mL) was deposited on the glass slides and allowed to react for at least 30 min at room temperature. Then, glass slides were washed 2 times in 1:1 ethanol:H₂O and rinsed in ultrapure water. In this way, glass slides were equipped with thiol groups as coupling groups on their surface. In order to bind peptides covalently to the surface, all peptides were modified with maleimide groups at their C-terminus. SEQ ID NO: 2, SEQ ID NO: 3, SEQ ID NO: 4 and SEQ ID NO: 7 maleimide modified peptides were dissolved in ultrapure water to a final concentration of 1 mg/mL. Glass slides were incubated for at least one hour in solutions with the maleimide-modified peptides. The mechanism of thiol-maleimide reactions is described as a typical Michael-type addition. This results in the formation of a thioether bond (-C-S-C-) between the glass and the applied peptides. The glass slides having a covalently bound peptide of the invention were arranged in plastic culture vessels. In each well, iPSC in mTeSR (Stemcell technologies, Vancouver, Canada) medium were incubated for 24 h up to 72 h at 37°C in a 5 % CO₂ atmosphere without agitation. After 72 h OCT4 expression was analysed via real-time quantitative polymerase chain reaction The iPSC that grew on the covalently immobilized peptides SEQ ID NO: 3 and SEQ ID NO: 7 showed a similarly strong OCT4 expression as iPSC cultured on Matrigel for 72 h. Covalently bound peptide of SEQ ID NO: 4 and of SEQ ID NO: 2 result in cells expressing OCT4 at lower levels. (Fig. 3).

Fig. 3 shows expression of the pluripotency marker OCT4 after 72 h detected on iPSC. The iPSC were maintained for 72 h on glass with one of the four peptides (SEQ ID NO: 2, SEQ ID NO: 3, SEQ ID NO: 4, and SEQ ID NO: 7) covalently bound, or cultured on Matrigel ^{®}. The y-axis shows the relative expression of OCT4 of the iPSC. The x-axis shows the bound peptide ID NO.

### Example 3: Cultivation of iPSC on a hydrogel functionalized with peptides

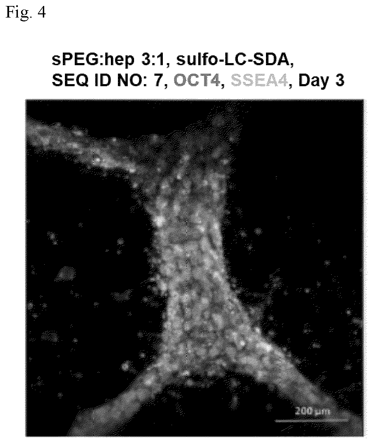

As a representative of a carrier which is a water-miscible polymer, a co-polymer of cross-linked PEG and heparin (sPEG-heparin) was provided with covalently bound peptides of one of SEQ ID NO: 1 to SEQ ID NO: 5, or with a covalently bound peptide of SEQ NO 7. In detail, functionalization was carried out by using sulfo-LC-SDA (sulfosuccinimidyl 6-(4,4'-azipentanamido)hexanoate) or sulfo-NHS (N-hydroxysulfosuccinimide) to covalently bind one of the different peptide sequences to this carrier. The iPSC show strong expression of the pluripotency markers OCT4 and SSEA4 even after 72 h cultivation on the functionalized hydrogel with SEQ ID NO: 7 (Fig.4). This shows that a hydrogel with a covalently bound peptide according to the invention is suitable for adherent growth of iPSC, while maintaining their pluripotency.

All steps were performed on ice. First, a co-polymer of PEG and heparin was washed 3 times with borate buffer (100 mM, pH 8.4) for 3 times (30 min). A solution of sulfo-LC-SDA (100 mM, 250 µl, dissolved in borate buffer) was added and the mixture was exposed to UV light (30 min). As the polymer backbones of both PEG and heparin contain -C-H and amino groups. The diazerin groups of sulfo-LC-SDA can initiate addition reactions with double bonds (C=C), insertion into C-H and N-H sites, or subsequent ring expansion to react as a nucleophile with primary amines. Consequently, sulfo-LC-SDA binds covalently to the hydrogel surface. The peptides were C-terminally derivatized by maleimide. The resultant hydrogels were washed three times with borate buffer (2 ml per well of 24 well plate) for one minute and laminin mixture/peptide solution were supplemented (laminin mixture: 70 µg/ml, 250 µl per well of 24 well plate, peptide solution: 1 mg/ml, 250 µl per well of 24 well plate). The samples were finally incubated at 4°C overnight and washed before seeding cells.

Fig. 4 shows an immunofluorescence image of iPSC on a sPEG-heparin hydrogel functionalized with the covalently bound peptide SEQ ID NO: 7. The hydrogel has a ratio of sPEG to heparin of 3:1 and was functionalized with the peptide SEQ ID NO: 7 (ASKAIQVFLLGG) via a sulfo-LC-SDA spacer. After 72 h cultivation, the pluripotency markers OCT4 (red) and SSEA-4 (green) were stained by immunofluorescence. The cell nuclei were stained with DAPI (blue) (scale bar 200 µm). This result shows that a peptide of the invention when covalently bound to a carrier, which can be a water-insoluble polymer, results in adherent growth of iPSC, e.g. for at least 24 h, preferably for at least 2d, e.g. for up to 5d.

## Claims

**1.** Cultivation process for cultivating induced pluripotent stem cells (iPSC) adherently on a carrier, comprising steps of providing a carrier having on its surface at least one covalently bound peptide, contacting a serum-free cultivation medium and iPSC with the carrier and incubating the carrier which has at least one covalently bound peptide in contact with the cultivation medium and iPSC under cell culture conditions, **characterized in that** the peptide consists of an amino acid sequence of 12 to 17 amino acids and has a section of at least 12 amino acids which is identical to a section of a peptide having one of SEQ ID NO: 2, SEQ ID NO: 3, SEQ ID NO: 4 as well as SEQ ID NO: 6 or its shorter version SEQ ID NO: 7, or a combination of at least two or all of these, wherein the peptide has a spacer group which is covalently linked to the surface of the carrier or the peptide is directly covalently bound to the carrier.

**2.** Cultivation process according to the preceding claim, **characterized in that** the carrier is a solid surface of a cultivation vessel, and the cultivation medium and iPSC are contacted with the solid surface by introducing the cultivation medium and iPSC into the cultivation vessel.

**3.** Cultivation process according to one of the preceding claims, **characterized in** incubating under static cell culture conditions.

**4.** Cultivation process according to one of the preceding claims, **characterized in that** the spacer is C-terminally bound to the peptide.

**5.** Cultivation process according to one of the preceding claims, **characterized in that** the carrier is a solid surface having covalently bound thioether groups to which maleimide modified laminin peptides are covalently bound.

**6.** Cultivation process according to one of the preceding claims, **characterized in that** the carrier is glass or plastics, and **in that** the carrier on its surface has a covalently bound group that is covalently bound to the spacer.

**7.** Cultivation process according to one of the preceding claims, **characterized in that** the carrier is a hydrogel.

**8.** Cultivation process according to one of the preceding claims, **characterized in that** the peptides are C-terminally covalently coupled to the carrier via functional groups at the C-terminus of the peptides such as maleimide and one, two or three C-terminally bound lysine residues and using coupling chemistry including e.g. sulfo-LC-SDA or sulfo-NHS/EDC.

**9.** Cultivation process according to one of the preceding claims, **characterized in that** exclusively iPSC are contained in the cultivation medium.

**11.** Process for producing a carrier for use in a cultivation process of iPSC according to one of the preceding claims, comprising the step of binding a peptide to a carrier, **characterized in that** the carrier consists of glass, of plastics or of a hydrogel, the carrier on its surface has covalently bound peptides, the peptides consisting of at least one amino acid sequence of 12 to 17 amino acids and having a section of at least 12 amino acids which is identical to one of SEQ ID NO: 2 to SEQ ID NO: 4 or having a section of at least 12 amino acids which is identical to SEQ ID NO: 7, wherein the peptide has a spacer group which is covalently linked to the surface of the carrier or the peptide is directly covalently bound to the carrier.

**12.** Process according to claim 11, wherein the peptide has a C-terminal spacer group which is reactive to form a covalent bond with the carrier.

**13.** Process according to claim 11 or 12, **characterized in that** the carrier has a covalently bound coupling group and **in that** the C-terminal spacer group is reactive to form a covalent bond with the coupling group.

**14.** Process according to one of claims 11 to 13, **characterized in that** the carrier or a coupling group covalently bound to the carrier has a thiol group.
